# EUROPÄISCHE PATENTANMELDUNG

(11) **EP 2 181 620 A1**
(43) Veröffentlichungstag der Anmeldung: **05.05.2010**
(21) Anmeldenummer: 08105693.9
(22) Anmeldetag: 29.10.2008
(51) Int. Cl.: A45D 40/26, A61M 35/00

(54) **Applikator und Verfahren zum Herstellen eines Applikators**

(71) Anmelder: Rondo AG, 4123 Allschwil (CH)
(72) Erfinder: Staub, Markus, 5703, Seon (CH); Schneider, Peter, 4106 Therwil (CH); Kälin, Alfred, 4310, Rheinfelden (CH); Krause, Martin, 68730, Blotzheim (FR)
(74) Vertreter: Wenger, René

(57) **Zusammenfassung**

Ein Spatel zum portionenweisen Dosieren von Heilmitteln wie Salben oder anderen pastösen Massen weist einen Basisabschnitt (2) und einen Deckabschnitt (3) aus einem transparenten Material auf. Der Deckabschnitt ist am Basisabschnitt verklebt. Zwischen dem Deckabschnitt (3) und dem Basisabschnitt (2) ist eine Fläche (4) mit einer Dosieranordnung (5) zum Dosieren der pastösen Massen aufgedruckt.

## Beschreibung

Die Erfindung betrifft einen Applikator zum Handhaben von pharmazeutischen oder kosmetischen Produkten. Derartige Applikatoren können beispielsweise Spatel sein, mit denen Heilmittel in Form von Salben oder andere pastösen Massen portioniert werden können. Weiterhin betrifft die Erfindung ein Verfahren zum Herstellen solcher Applikatoren.

Als Applikatoren bekannt und gebräuchlich sind Spatel, die einstückig ausgestaltet sind und aus Kunststoff oder Holz bestehen. Sie haben den Nachteil, dass der Materialverbrauch verhältnismässig gross ist und sie damit verhältnismässig teuer sind.

Bei der Anwendung von Heilmitteln spielt die Dosierung eine entscheidende Rolle. Dies gilt zum Beispiel für Salben mit hochwirksamen Wirkstoffen, die genau dosiert werden müssen. Vorstellbar hierzu sind Spatel, deren Oberseite mit einer Skalierung bedruckt sind. Zum portionenweisen Dosieren muss der Anwender den Tubeninhalt entlang einer durch die Skala vorgegebene Dosierstrecke bis auf eine gewünschte Länge herausdrücken. Da das Heilmittel direkt auf die bedruckte Fläche gelangt, kann es zu Verunreinigungen des Heilmittels durch Druckfarbe kommen.

Es ist deshalb eine Aufgabe der vorliegenden Erfindung die Nachteile des Bekannten zu vermeiden und insbesondere einen Applikator zum Handhaben von pharmazeutischen oder kosmetischen Produkten zu schaffen, welcher einfach und kostengünstig herstellbar ist. Weiterhin soll der Applikator beschriftbar sein, ohne dass unerwünschte Verunreinigungen der auf den Applikator aufgetragenen Produkte durch die Anzeigemittel stattfinden können. Diese Aufgaben werden mit einem Applikator gelöst, der die Merkmale von Anspruch 1 aufweist.

Der Applikator enthält einen Basisabschnitt und einen Deckabschnitt aus einem vorzugsweise transparenten oder teiltransparenten Material. Der Basisabschnitt kann aus Kunststoff bestehen. Selbstverständlich sind aber auch andere Materialen für den Basisabschnitt (z.B. Aluminium oder Alumiumlegierungen) vorstellbar. Der Deckabschnitt kann eine Oberseite des Applikators vorgeben, auf welche die erwähnten Produkte applizierbar oder auftragbar sind. Der Deckabschnitt kann beispielsweise durch eine transparente Folie aus Kunststoff gebildet sein. Vorteilhaft besteht der Deckabschnitt aus einem chemisch beständigen Material, das gegen die Einwirkung des Produkts widerstandsfähig ist. Insbesondere soll das Material vorzugsweise wasser- und/oder schmutzabweisende Eigenschaften aufweisen. Dadurch, dass der Deckabschnitt vollständig oder teilweise am Basisabschnitt befestigt ist, kann für den Basisabschnitt ein verhältnismässig kostengünstiges Material gewählt werden. Die Materialkosten können somit gesenkt werden.

Als Applikatoren kommen unter anderem Spatel zum portionenweisen Dosieren von Heilmitteln wie Salben oder anderen pastösen Massen in Frage. Die Heilmittel können beispielsweise in Tuben oder Spritzen abgefüllt sein. Als Applikatoren sind aber auch Dosierlöffel oder andere Hilfsmittel zum Verabreichen von Verpackungsgut (wie etwa Pulver, Pasten, Flüssigkeiten) denkbar.

Der Applikator zum Handhaben von pharmazeutischen oder kosmetischen Produkten eignet sich grundsätzlich aber auch für andere Anwendungsgebiete. So kann der Applikator auch im Veterinärbereich oder im Agrarbereich eingesetzt werden. Beispielsweise kann der Applikator ein Mess- oder Dosierlöffel für Pflanzenschutzmittel sein.

Vorteilhaft kann es sein, wenn der Deckabschnitt den Basisabschnitt auf einer Seite vollständig abdeckt. Diese Seite kann eine Oberseite bilden, auf die die pharmazeutischen oder kosmetischen Produkte applizierbar sind. Theoretisch ist es aber auch denkbar, dass ein flächiger Basisabschnitt auf jeder Seite abgedeckt ist, wodurch sowohl die Vorderseite (Oberseite) als auch die Rückseite des Applikators als Applikationsbereiche einsetzbar sind.

Der Deckabschnitt kann mit dem Basisabschnitt verklebt oder verschweisst sein. Selbstverständlich wären aber auch andere Befestigungsarten vorstellbar.

Eine vorteilhafte Handhabung kann sich dadurch ergeben, wenn zwischen dem Deckabschnitt und dem Basisabschnitt eine Fläche mit Anzeigemitteln zum Applizieren der Produkte angeordnet ist. Diese Anzeigemittel können einen Anwender dahingehend instruieren, welche Menge des Heilmittels er auf den Applikator aufbringen darf. Diese Anordnung stellt sicher, dass die Heilmittel nicht direkt mit der Fläche mit den Anzeigemitteln in Kontakt kommen.

Der Basisabschnitt kann mit Applikationsanweisungen und/oder Dosieranordnungen bedruckt sein. Die bedruckte Fläche ist dabei zwischen dem Deckabschnitt und dem Basisabschnitt angeordnet. Durch die Verwendung von transparentem Material für den Deckabschnitt ist die bedruckte Fläche durch den Deckabschnitt für den Anwender einsehbar. Eine Dosieranordnung kann beispielsweise eine Skala sein, die eine Dosierstrecke zum Abmessen von Pasten oder Gels aus Tuben oder Spritzen vorgibt. Selbstverständlich ist es aber auch vorstellbar, dass der Basisabschnitt mit anderen Anzeigemitteln bedruckt ist.

Der Applikator kann aus zwei Lagen (Basisabschnitt, Deckabschnitt) bestehen. Vorteilhaft kann es aber auch sein, wenn der Applikator mehr als nur zwei Schichten aufweist. Beispielsweise kann der Applikator zusätzlich einen Zwischenabschnitt aufweisen, der zwischen dem Bodenabschnitt und dem Deckabschnitt angeordnet oder sandwichartig aufgenommen ist.

Der Zwischenabschnitt kann zum Versiegeln gas- und/oder flüssigkeitsdicht zwischen dem Bodenabschnitt und dem Deckabschnitt aufgenommen sein.

Weiterhin kann es vorteilhaft sein, wenn der Zwischenabschnitt aus Papier, Karton oder einem Kartonlaminat besteht. Derartige Zwischenabschnitte haben den Vorteil, dass sie einfach mit Anzeigemitteln versehen (z.B. bedruckt oder auf andere Weise beschriftet) werden können.

Besonders vorteilhaft kann es sein, wenn auf dem Zwischenabschnitt eine Skala oder eine andere Dosieranordnung zum Dosieren von Salben oder anderen pastösen Massen angebracht und insbesondere aufgedruckt ist.

Der Basisabschnitt kann wenigstens eine vorzugsweise durch Tiefziehoperationen geschaffene rinnenförmige Vertiefung aufweisen. Die rinnenförmige Vertiefung kann eine Senke zum Stabilisieren des Applikators bilden. Die rinnenförmige Vertiefung kann sich in linearer Richtung erstrecken. Selbstverständlich ist es aber auch vorstellbar, - anstatt einer geraden Rinne - eine gekrümmte Rinne vorzusehen. Die Vertiefung kann einen Hohlraum zur Aufnahme des Zwischenabschnitts bilden.

Ein weiterer Aspekt der Erfindung betrifft eine Anordnung mit einer Faltschachtel aus Karton oder aus Kartonlaminat in die Verpackungsgut wie Tuben, Dosen, Fläschchen, Ampullen oder andere Gegenstände aufgenommen oder aufnehmbar ist. Die Anordnung enthält weiter den vorgängig beschriebenen Applikator zum Handhaben von Produkten, die im genannten Verpackungsgut enthalten sind.

Besonders vorteilhaft kann es sein, wenn der Applikator vorzugsweise lösbar in oder an der Faltschachtel befestigt ist. Der Applikator kann also auf einer Innenseite oder auf einer Aussenseite einer Seitenwand der Faltschachtel fixiert oder an diese fixierbar sein. Die Faltschachtel kann aus einem einzigen Zuschnitt hergestellt werden, wobei der Zuschnitt nach Falz- und Klebeoperationen einen quaderförmigen Verpackungskörper bilden kann. Zum lösbaren Fixieren kann die entsprechende Innenseite wenigstens abschnittsweise mit einem Klebstoff, insbesondere mit einem Haftklebstoff beschichtet sein. Durch die Verwendung von Haftklebstoff ist eine vorteilhafte lagemässige Fixierung des Applikators sichergestellt. Alternativ können auch Perforationslinien derart in der Faltschachtel und/oder im Applikator angeordnet sein, durch welche der Applikator auf einfache Art und Weise wegreissbar bzw. aus der Faltschachtel entnehmbar ist.

In verfahrensmässiger Hinsicht ist es zweckmässig, wenn zum Vorgeben wenigstens eines Basisabschnitts ein flächiges Material bereitgestellt wird und ein Deckabschnitt aus einem vorzugsweise transparenten oder teiltransparenten Material auf den Basisabschnitt vorzugsweise durch Kleben befestigt wird.

Eine rationelle Fertigung kann sich dadurch ergeben, wenn zum Vorgeben einer Mehrzahl von Applikatoren ein flächiges Material für die Basisabschnitt und eine Folie für die Deckabschnitte bereitgestellt wird. Die Folie kann dann am flächigen Material befestigt (z.B. verklebt) werden. Diese wenigstens zweilagige Anordnung kann schliesslich vorzugsweise durch Stanzoperationen derart bearbeitet werden, dass die Applikatoren vereinzelt werden können.

Besonders vorteilhaft kann es sein, wenn für die Herstellung der Applikatoren konventionelle Anlagen zur Zerstellung von Blisterverpackungen oder -streifen verwendet werden. Diese Anlagen können durch Modifikationen einfach umgerüstet werden.

Weiterhin kann es vorteilhaft sein, wenn durch Tiefziehen eine vorzugsweise rinnenförmige Vertiefung in den Basisabschnitt oder die Basisabschnitte eingebracht wird, Wenn in die Vertiefung ein Zwischenabschnitt aus Papier, Karton oder ein Kartonlaminat abgelegt wird und wenn der Zwischenabschnitt beim Befestigen des Deckabschnitts am Basisabschnitt versiegelt wird.

Weitere Vorteile und Ausgestaltungen der Erfindung ergeben sich aus der nachstehenden Beschreibung von Ausführungsbeispielen und aus den Zeichnungen. Es zeigen:
- Figur 1: eine Draufsicht auf einen Applikator in Form eines Spatels zum portionenweisen Dosieren von Salben,
- Figur 2: eine Querschnittsdarstellung des Applikators aus Fi- gur 1,
- Figur 3: das Detail A aus Figur 2 in vergrösserter Darstel- lung,
- Figur 4: der Applikator aus Figur 1 während einem Dosiervor- gang,
- Figur 5: eine Draufsicht auf einen alternativen Applikator,
- Figur 6: der Applikator aus Figur 5 während einem Dosiervor- gang,
- Figur 7: eine perspektivische Explosionsdarstellung eines Ap- plikators gemäss einem dritten Ausführungsbeispiel,
- Figur 8: eine Draufsicht auf einen weiteren alternativen Ap- plikator, und
- Figur 9: ein flächiges Material für die Bildung einer Mehrzahl von Applikatoren.

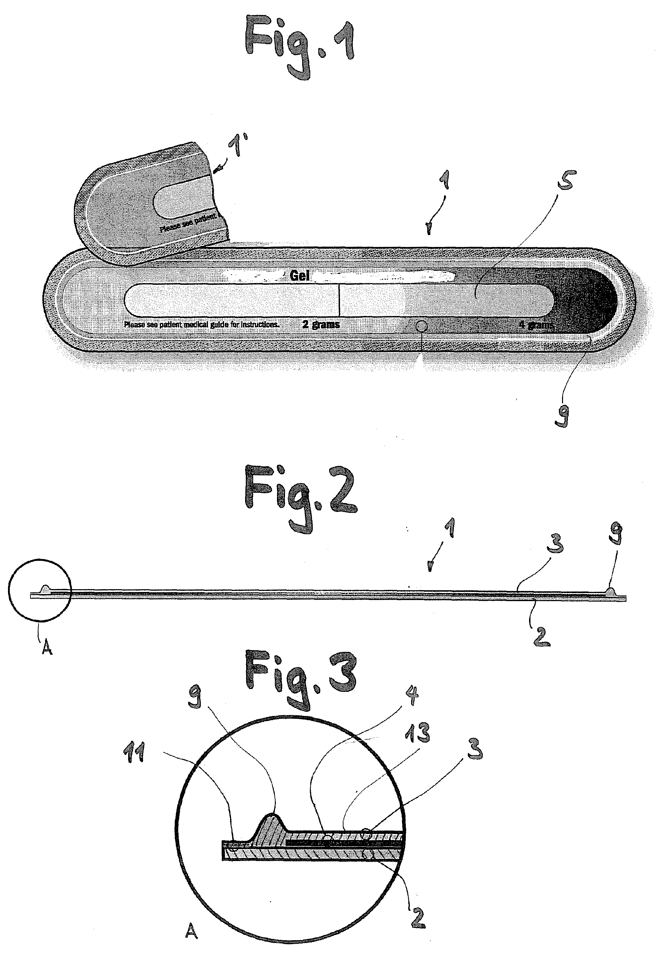

Figur 1 zeigt einen mit 1 bezeichneten Applikator zum Handhaben von pharmazeutischen oder kosmetischen Produkten. Mit 1' ist ein Teil eines zweiten Applikators erkennbar. Die vorliegenden Applikatoren sind als Spatel ausgestaltet, die zum Dosieren von Salben, Gels oder andern pastösen Massen verwendet werden können. Mit 5 sind Anzeigemittel zum Applizieren des entsprechenden Produktes angedeutet. Die Anzeigemittel enthalten unter anderem eine Skala mit einer Dosierstecke, mit deren Hilfe wahlweise zwei oder vier Gramm eines Gels auf den Spatel 1 aufgetragen werden können. Weiter ist in Figur 1 eine in der Draufsicht umlaufende Erhebung 9 erkennbar. Der genaue Aufbau des Applikators wird nachfolgend anhand der Figuren 2 und 3 im Detail erläutert.

Wie Figur 2 zeigt, ist der Spatel zweilagig aufgebaut. Die erste Lage wird dabei durch einen Basisabschnitt 2 gebildet (Rückseite). Die zweite Lage wird durch einen Deckabschnitt 3 gebildet (Vorderseite), der am Basisabschnitt 2 befestigt ist. Wie aus der Detaildarstellung gemäss Figur 3 hervorgeht, befindet sich zwischen den beiden Lagen, d.h. zwischen dem Basisabschnitt 2 und dem Deckabschnitt 3, eine mit 4 bezeichnete Fläche mit den vorgängig erwähnten Anzeigemitteln. Die Fläche 8 kann beispielsweise bedruckt sein.

Aus Figur 3 geht hervor, dass der Basisabschnitt 2 und der Deckabschnitt 3 an den Seitenrändern miteinander verklebt sind. Die dadurch entstehende Versiegelung ist mit 11 angedeutet. Die Deckschicht bildet eine Art Schutzschicht für die mit Anzeigemitteln versehene Fläche 4. Wird ein Produkt auf die Oberseite 13 des Applikators aufgetragen, so kann das Produkt nicht in Kontakt mit dem Anzeigemittel kommen. Dadurch kann eine unerwünschte Kontamination z.B. mit Druckfarbe ausgeschlossen werden kann.

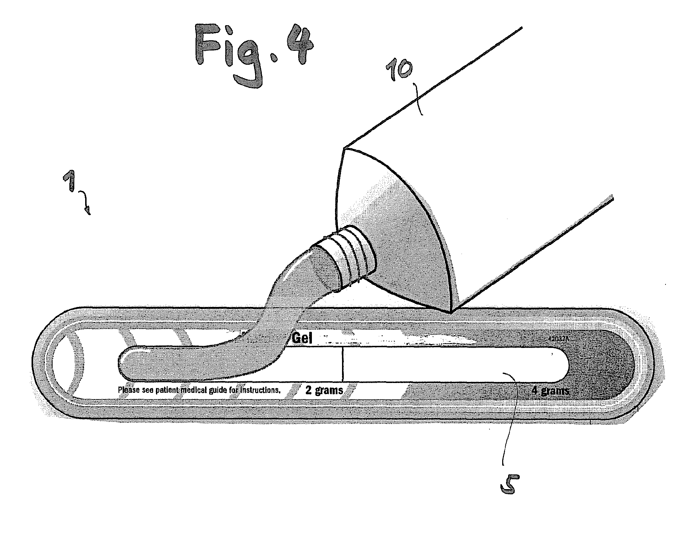

Figur 4 zeigt einen möglichen Anwendungsbereich des Spatels 1. Zum Dosieren einer Salbe oder eines Gels aus einer Tube 10 muss der Anwender einen Produktestrang in der gewünschten oder vorgeschriebenen Länge (hier beispielhaft: 2 oder 4 Gramm) auf den Applikator 1 auftragen.

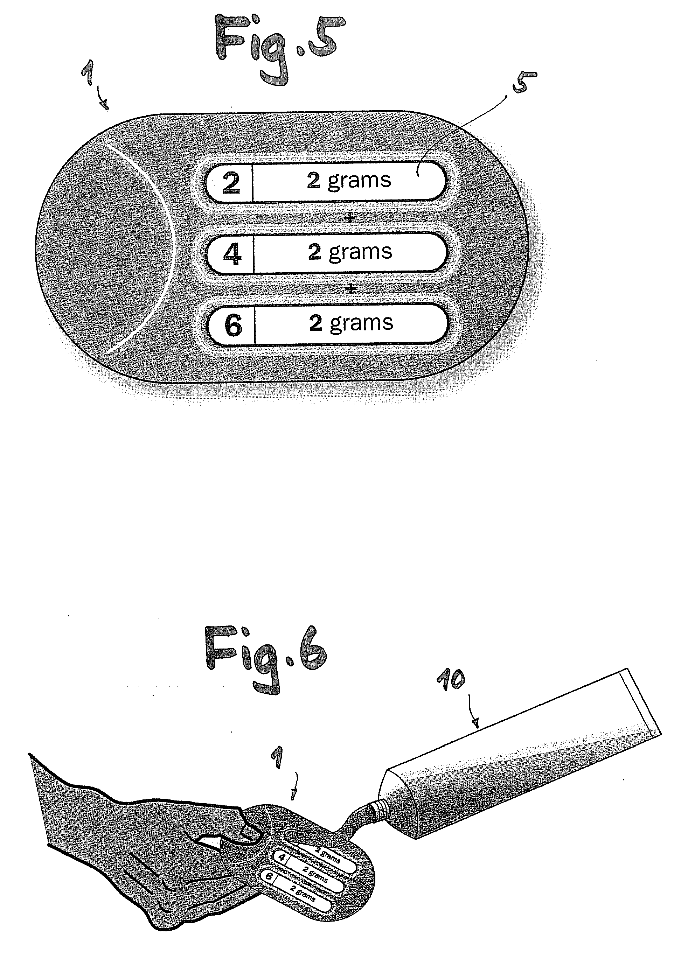

Die Figuren 5 und 6 zeigen einen weiteren Applikator 1. Neben einer leicht veränderten Form unterscheidet sich der Applikator 1 vom Applikator gemäss dem vorhergehenden Ausführungsbeispiel vor allem durch eine andere Gestaltung der Dosieranordnung. Anstatt einer zusammenhängenden Dosierstrecke weist er einzelne Dosierstrecken für die pastösen Massen (je beispielhaft zwei Gramm) auf. Selbstverständlich sind aber auch andere Anzeigemittel vorstellbar.

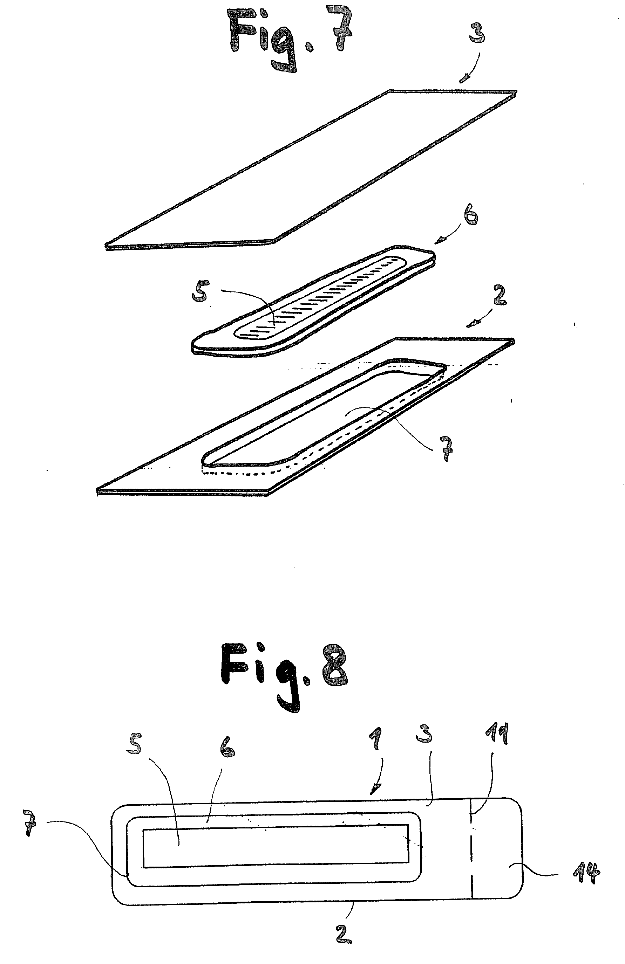

Figur 7 zeigt eine dreilagige Ausführungsform eines Applikators. Neben dem Basisabschnitt 2 und dem Deckabschnitt 3 kommt zusätzlich ein Zwischenabschnitt 6 hinzu, der sandwichartig zwischen den beiden äussern Lagen aufnehmbar ist. Der Zwischenabschnitt 6 besteht aus Papier, Karton oder einem Kartonlaminat. Im Basisabschnitt 2 ist eine durch einen Tiefziehvorgang geschaffene Vertiefung 7 erkennbar, in die der Zwischenabschnitt 6 ablegbar ist. Der Zwischenabschnitt 6 ist mit einer Skala 5 bedruckt.

Figur 8 zeigt eine Draufsicht auf eine Oberseite eines Applikators 1, der ebenfalls dreilagig ausgestaltet ist. Durch den transparenten Deckabschnitt ist der in der Vertiefung 7 positionierte Zwischenabschnitt 6 für den Anwender einsehbar. Eine Besonderheit dieses Applikators 1 besteht darin, dass eine Perforationslinie 11 im Basisabschnitt 2 angeordnet ist. Rechts der Perforationslinie 11 ist ein mit 14 bezeichnetes Segment erkennbar, das an eine (nicht gezeigte) Faltschachtel befestigt werden kann. Damit ist der in oder an der Schachtel fixierte Applikator auf einfache Art und Weise wegreissbar oder aus der Faltschachtel entnehmbar. Der Deckabschnitt 3 deckt bei diesem Ausführungsbeispiel den Basisabschnitt 2 auf der Oberseite nicht vollständig ab, sondern überlappt diesen nur bis zur Perforationslinie 11. Das Segment 14 des Basisabschnitts 2 ist also nicht vom Deckabschnitt 3 abgedeckt. Damit entsteht ein Bereich 14, der auf vorteilhafte Art und Weise als Klebefläche genutzt werden kann.

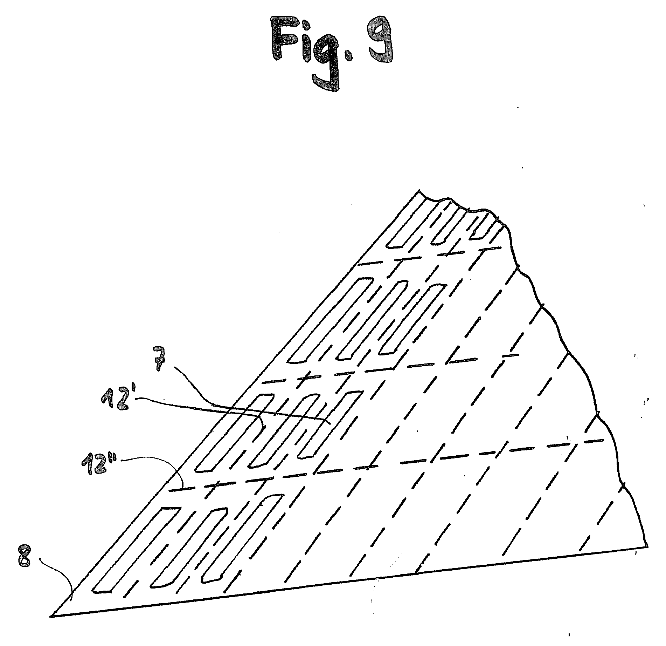

Anhand Figur 9 ist erkennbar wie eine Mehrzahl von Applikatoren auf einfache Art und Weise hergestellt werden können. Zum Vorgeben einer Mehrzahl von Applikatoren wird zunächst ein flächiges Material 8 für die Basisabschnitte bereitgestellt. Auf dieses flächige Material kann dann eine Folie für die Deckabschnitte befestigt werden. Anschliessend können dann durch Stanzoperationen die Applikatoren 1 vereinzelt werden. Die mit 12' und 12" bezeichneten und gestrichelten Linien deuten (imaginäre) Schnittlinien an, nach welchen zum Vereinzeln der Applikatoren gestanzt werden soll. Auf dem flächigen Material 8 befinden sich rinnenförmige Vertiefungen 7, die - vor dem Vereinzelungsschritt und vorzugsweise vor dem Befestigen einer (hier nicht gezeigten) Folie für die Deckabschnitte - durch Tiefziehen eingebracht wurden.

Zum Herstellen der Applikatoren können Anlagen verwendet werden, mit denen Blister zum Abpacken von Tabletten, Kapseln etc. gefertigt werden können. Derartige Blistermaschinen oder -anlagen werden zum Beispiel unter der Bezeichnung "MediSeal®" vertrieben. Handelsübliche Blistermaschinen der Firma MediSeal GmbH, Schloss Holte, Deutschland tragen die Typenbezeichnungen "CP200", "CP400", "CP600", "CP1200", "CP2", "CP3" oder "CP10/11". Mit solchen oder ähnlichen Blistermaschinen sind die Arbeitsschritte zum Herstellen der Applikatoren wie Formen von Vertiefungen, Ablegen von Zwischenabschnitten (optional), Befestigen und Versiegeln sowie Stanzen auf einer Anlage auf vorteilhafte Art möglich.

## Patentansprüche

1. Applikator zum Handhaben von pharmazeutischen oder kosmetischen Produkten, insbesondere Spatel zum portionenweisen Dosieren von Heilmitteln wie Salben oder anderen pastösen Massen, enthaltend einen Basisabschnitt (2) und einen Deckabschnitt (3) aus einem vorzugsweise transparenten oder teiltransparenten Material, wobei der Deckabschnitt wenigstens teilweise am Basisabschnitt befestigt ist.

2. Applikator nach Anspruch 1, **dadurch gekennzeichnet, dass** der Deckabschnitt (3) den Basisabschnitt (2) auf einer Seite vollständig abdeckt.

3. Applikator nach Anspruch 1 oder 2, **dadurch gekennzeichnet, dass** der Deckabschnitt (3) mit dem Basisabschnitt (2) verklebt oder verschweisst ist.

4. Applikator nach einem der Ansprüche 1 bis 3, **dadurch gekennzeichnet, dass** zwischen dem Deckabschnitt (3) und dem Basisabschnitt (2) eine Fläche (4) mit Anzeigemitteln (5) zum Applizieren der Produkte angeordnet ist.

5. Applikator nach einem der Ansprüche 1 bis 4, **dadurch gekennzeichnet, dass** der Basisabschnitt (2) mit Applikationsanweisungen und/oder Dosieranordnungen (5) bedruckt ist.

6. Applikator nach einem der Ansprüche 1 bis 5, **dadurch gekennzeichnet, dass** zwischen dem Basisabschnitt (2) und dem Deckabschnitt (3) ein Zwischenabschnitt (6) angeordnet ist.

7. Applikator nach Anspruch 6, **dadurch gekennzeichnet, dass** der Zwischenabschnitt (6) zum Versiegeln gas- und/oder flüssigkeitsdicht zwischen dem Basisabschnitt (2) und dem Deckabschnitt (3) aufgenommen ist.

8. Applikator nach Anspruch 6 oder 7, **dadurch gekennzeichnet, dass** der Zwischenabschnitt (6) aus Papier, Karton oder einem Kartonlaminat besteht.

9. Applikator nach einem der Ansprüche 6 bis 8, **dadurch gekennzeichnet, dass** auf dem Zwischenabschnitt (6) eine Skala (5) oder eine andere Dosieranordnung zum Dosieren von Salben oder anderen pastösen Massen angebracht und insbesondere aufgedruckt ist.

10. Applikator nach einem der Ansprüche 1 bis 9, **dadurch gekennzeichnet, dass** der Basisabschnitt (2) aus Kunststoff besteht.

11. Applikator nach einem der Ansprüche 1 bis 10, **dadurch gekennzeichnet, dass** der Basisabschnitt (2) wenigstens eine vorzugsweise durch Tiefziehoperationen geschaffene rinnenförmige Vertiefung (7) aufweist.

12. Applikator nach Anspruch 11, **dadurch gekennzeichnet, dass** die Vertiefung (7) einen Hohlraum zur Aufnahme des Zwischenabschnitts (6) bildet.

13. Anordnung mit einer Faltschachtel aus Karton oder aus Kartonlaminat, in die Verpackungsgut wie Tuben, Dosen, Fläschchen, Ampullen, oder anderen Gegenstände aufgenommen oder aufnehmbar ist und mit einem Applikator (1) zum Handhaben der im Verpackungsgut enthaltenen pharmazeutischen oder kosmetischen Produkten gemäss einem der Ansprüche 1 bis 12.

14. Verfahren zum Herstellen eines Applikators (1) zum Handhaben von pharmazeutischen oder kosmetischen Produkten, insbesondere eines Spatels zum Dosieren von Salben oder anderen pastösen Massen insbesondere nach einem der Ansprüche 1 bis 12, bei dem zum Vorgeben wenigstens eines Basisabschnitts (2) ein flächiges Material bereitgestellt wird und ein Deckabschnitt (3) aus einem vorzugsweise transparenten oder teiltransparenten Material auf den Basisabschnitt (2) vorzugsweise durch Kleben befestigt wird.

15. Verfahren nach Anspruch 14, **dadurch gekennzeichnet, dass** zum Vorgeben einer Mehrzahl von Applikatoren (1) ein flächiges Material (8) für die Basisabschnitte (2) und eine Folie für die Deckabschnitte (3) bereitgestellt wird, dass die Folie am flächigen Material (8) befestigt wird und dass anschliessend vorzugsweise durch Stanzoperationen die Applikatoren (1) vereinzelt werden.

16. Verfahren nach Anspruch 14 oder 15, **dadurch gekennzeichnet, dass** durch Tiefziehen eine vorzugsweise rinnenförmige Vertiefung (7) in den Basisabschnitt (2) eingebracht wird, dass in die Vertiefung ein Zwischenabschnitt (6) aus Papier, Karton oder einem Kartonlaminat abgelegt wird und dass der Zwischenabschnitt (6) beim Befestigen des Deckabschnitts (3) am Basisabschnitt (2) versiegelt wird.
